# EUROPEAN PATENT SPECIFICATION

(11) **EP 2 040 677 B1**
(45) Date of publication and mention of the grant of the patent: **08.05.2019**
(21) Application number: 07765733.6
(22) Date of filing: 29.06.2007
(51) Int. Cl.: A61K 9/16, A61K 31/496

(54) **IMPROVEMENTS RELATING TO ANTI-PARASITIC COMPOSITIONS**
VERBESSERUNGEN IM ZUSAMMENHANG MIT ANTIPARASITEN-ZUSAMMENSETZUNGEN
COMPOSITIONS ANTIPARASITAIRES AMÉLIORÉES

(30) Priority: 13.07.2006 GB 0613925
(43) Date of publication of application: 01.04.2009
(73) Proprietor: The University of Liverpool, Liverpool, L69 7ZX (GB)
(72) Inventor: DUNCALF, David, John, Little Neston Wirral CH64 0TU (GB); ESSA, Asha, Hassan, Liverpool L8 2XP (GB); FOSTER, Alison, Jayne, Wirral Merseyside CH63 8QB (GB); LONG, James, Wirral CH43 1TF (GB); RANNARD, Steven, Paul, Bebington, Wirral Merseyside CH63 3JW (GB); WANG, Dong, Liverpool Merseyside L69 7ZB (GB)
(74) Representative: Cawley, Aimee Elizabeth
(86) International application number: PCT/EP2007/056561
(87) International publication number: WO 2008/006713

(56) References cited:
- EP-A- 1 080 720
- WO-A-02/09667
- WO-A-2004/075921
- VAN NIJLEN T ET AL: "Improvement of the dissolution rate of artemisinin by means of supercritical fluid technology and solid dispersions." INTERNATIONAL JOURNAL OF PHARMACEUTICS (KIDLINGTON), vol. 254, no. 2, 26 March 2003 (2003-03-26), pages 173-181, XP002477067 ISSN: 0378-5173
- BODMEIER R ET AL: "Polylactic acid microspheres containing quinidine base and quinidine sulphate prepared by the solvent evaporation technique. II. Some process parametersinfluencing the preparation and properties of micropheres" JOURNAL OF MICROENCAPSULATION, TAYLOR AND FRANCIS, BASINGSTOKE, GB, vol. 4, 1987, pages 289-297, XP009089876 ISSN: 0265-2048
- KAYSER O ET AL: "FORMULATION AND BIOPHARMACEUTICAL ISSUES IN THE DEVELOPMENT OF DRUG DELIVERY SYSTEMS FOR ANTIPARASITIC DRUGS" PARASITOLOGY RESEARCH, SPRINGER VERLAG, BERLIN, DE, vol. 90, no. SUPPL 2, 1 June 2003 (2003-06-01), pages S63-S70, XP008039883 ISSN: 0932-0113

## Description

### Field of the Invention

The present invention relates improvements relating to anti-malarial compositions.

In particular it relates to pharmaceutically active compositions and precursors therefor which contains a substance active against malaria parasites and which in particular has a high activity against multi-resistant lines of malaria parasites, particularly ***Plasmodium falciparum.***

The present invention further relates to a pharmaceutically acceptable form of artemisinin and/or a derivative of artemisinin, for example arteether, artemether, artemisinin, dihydroartemisinin or artesunate, whether alone or in combination with another anti-malarial agent.

### Background of the Invention

Malaria is a vector-borne infectious disease that is widespread in tropical and subtropical regions. It infects between 300 and 500 million people every year and causes between one and three million deaths annually, mostly among young children in Sub-Saharan Africa. The disease is caused by protozoan of the genus Plasmodium. The most serious forms of the disease are caused by ***P. falciparum*** and ***P. vivax*** but other related species ***P. ovale, P. malariae*** and ***P. knowlesi*** can infect humans. This group of human-pathogenic *Plasmodium* species is usually referred to as the *malaria parasites.* Many areas of the world now have widespread strains of chloroquine-resistant ***P*. *falciparum.***

Effective agents for the treatment and prophylaxis of malaria have been known for many years however there has been a long standing problem that these have a poor water solubility. A well known solution to this problem has been to dissolve the anti-malarial agent in alcohol and consume the solution. Thus quinine could be dissolved in gin (by the English) and artemisinin used to make absinthe (by the French). While these were popular treatments, they were not without side-effects due to the levels of alcohol used and could not be used by abstainers from alcohol.

Artemisinin (qinghaosu) is obtained from the leaves of the shrub ***Artemisia annua*** and is a naturally occurring sesquiterpene lactone with an endo peroxide group. *Artemisia* has been used by Chinese herbalists for more than a thousand years in the treatment of many illnesses, including malaria. Its mechanism of action is not fully understood. It is also believed to be effective in the treatment of other parasitic infections including those caused by worms and flukes.

Artemisinin preparations are currently among the substances which are believed to act most rapidly against malaria parasites. In particular, they show a high activity against multiresistant lines of ***Plasmodium falciparum.*** Also, when administered to humans, only a few side effects and no significant toxicity have been observed, although neurotoxicity has occurred in animals.

However, physical properties such as poor water solubility (in the case of artemisinin, artemether, arteether, dihydroartemisinin), poor bioavailability (in the case of artemisinin, artemether, arteether), or poor stability (in the case of artesunate) often limit their effectiveness, application, and increase their cost either by having to increase dosages or by using special packaging. Artemisinin is therefore mostly presented in a tablet form. For example U.S. Pat. Nos. 6,326,023; 6,307,068; 6,306,896; 5,834,491; 5,677,331; 5,637,594; 5,486,535; 5,278,173; 5,270,037; 5,219,865; 5,021,426; 5,011,951 are only directed to compositions which are to be packaged as solids for oral dosing.

Artemisinin is typically used in treatment of malaria rather than in prophylaxis. Malarial patients in the advanced stages of the disease may be unable to swallow tablets.

Therefore it is desirable to have anti-malarial drugs available in liquid form to provide an injectable medication or allow rectal administration. Liquid forms of medication also allow for a continuous variation in dosage and this is especially useful in the treatment of children, where dosage may be calculated on the basis of body weight.

Attempts have been made to improve the solubility of less soluble Artemisinin-type drugs so as allow liquid forms, but these can cause side-effects at the injection site or the formulation may introduce undesirable carrier materials (such as DMSO). US 7084132 describes many of these problems and offers a solution in the form of admixing the drugs with cyclodextrins.

It is therefore desirable to provide artemisinin-type drugs in a soluble form. Advantageously, these should form physiologically acceptable solutions. It is also advantageous that the drugs should exhibit high bioavailability.

Quinine and its stereoisomer quinidine, are rapidly acting schizontocides that target the erythrocytic asexual stages of all malaria parasites. Previously chloroquine was used instead of quinine, but it has now become the first choice of treatment due malarial resistance of chloroquine.

Application of an anti-malarial formulation must be specific to administration in hot, humid tropical regions native to the malarial parasites. Thus, chemical stability under drastic environmental conditions is desirable. It is therefore a further advantage that they should be storage stable.

The activity of artemisinin and its derivatives is shortlived when compared to other anti-malarial drugs. With significant decreases in effectiveness after one to two hours, artemisinin and its derivatives are administered in combination with long half-life anti-malarial drugs, such as lumefantrine, mefloquine, or amodiaquine, etc. to treat uncomplicated *falciparum* malaria.

Lumefantrine has a half-life of about 3 to 6 days. Such a treatment is called ACT (artemisinin-based combination therapy). Examples include artemether-lumefantrine, artesunate-mefloquine, artesunate-amodiaquine, and artesunate-sulfadoxine/ pyrimethamine. Trials have shown that ACT is more than 90% effective, with a recovery from malaria after three days, especially for the chloroquine-resistant ***P*.** ***falciparum.*** Advantageously, artemisinin should be presented in a form which makes it suitable for combination treatment.

Our co-pending international patent application PCT/GB03/03226 describes the formation of solid, porous beads comprising a three dimensional open-cell lattice of a water-soluble polymeric material. These are typically 'templated' materials formed by the removal of both water and a non-aqueous dispersed phase from a high internal phase emulsion (HIPE) which has a polymer dissolved in the aqueous phase. The beads are formed by dropping the HIPE emulsion into a low temperature fluid such as liquid nitrogen, then freeze-drying the particles formed to remove the bulk of the aqueous phase and the dispersed phase. This leaves behind the polymer in the form of a 'skeletal' structure. The beads dissolve rapidly in water and have the remarkable property that a water-insoluble component dispersed in the dispersed phase of the emulsion prior to freezing and drying can also be dispersed in water on solution of the polymer skeleton of the beads.

WO 2005/011636 discloses a non-emulsion based spray drying process for forming 'solid amorphous dispersions' of drugs in polymers. In this method a polymer and a low-solubility drug are dissolved in a solvent and spray-dried to form dispersions in which the drug is mostly present in an amorphous form rather than in a crystalline form.

Our co-pending applications GB 0501835 and GB 0613925 (filed 13th July 2006) describe how materials which will form a nano-dispersion in water can be prepared, preferably by a spray-drying process. In the first of these applications the water insoluble materials is dissolved in the solvent-phase of an emulsion. In the second, the water-insoluble materials are dissolved in a mixed solvent system and co-exist in the same phase as a water-soluble structuring agent. In both cases the liquid is dried above ambient temperature (above 20 Celsius), such as by spray drying, to produce particles of the structuring agent, as a carrier, with the water-insoluble materials dispersed therein. When these particles are placed in water they dissolve, forming a nano-dispersion of the water-insoluble material with particles typically below 300nm. This scale is similar to that of virus particles, and the water-insoluble material behaves as though it were in solution.

In the present application the term 'ambient temperature' means 20 degrees Celsius and all percentages are percentages by weight unless otherwise specified.

Our GB 0501835 application showed that fluorescer materials prepared by the method disclosed exhibited better performance than those prepared by a known freeze-drying method.

Our GB 0613925 application makes it clear that a Triclosan™ nano-dispersion has the additional benefit that weight for weight it is more effective than is normally expected of Triclosan™ even at very low concentrations.

### Brief Description of the Invention

We have now determined that both the emulsion-based and the single-phase method can be used to produce a water-soluble form of anti-malarial drugs, particularly relatively insoluble 'artemisinin-type' and 'quinine-type' anti-malarial drugs.

Accordingly, a first aspect of the present invention provides a composition comprising at least one water insoluble anti-malarial drug and da water-soluble carrier material,
wherein the water-insoluble anti-malarial drug is selected from artemisinin, arteether, artemether, dihydroartemisinin and artesunate, whether alone or in combination with another anti-malarial agent, or is selected from quinine and quinidine,
wherein said water-soluble carrier material comprises a polymer and a surfactant, the polymer being selected from polyvinylpyrrolidone (PVP), hydroxypropyl cellulose, hydroxypropyl methylcellulose (HPMC), and mixtures thereof, the surfactant being selected from alkoxylated nonionics, phenolethoxylates, alkyl sulphonates, ester surfactants, cationics, and mixtures thereof,
wherein the water-insoluble anti-malarial drug is dispersed through the carrier material in nano-disperse form having a peak diameter of the nano-disperse form within the composition of 20-800nm.

Also disclosed is an anti-malarial drug preparation comprising at least one water insoluble anti-malarial drug and a water-soluble carrier material, wherein the water-insoluble anti-malarial drug is dispersed through the carrier material in nano-disperse form having a peak diameter of the nano-disperse form of 20-800nm.

The preferred method of particle sizing for the dispersed products of the present invention employs a dynamic light scattering instrument (Nano S, manufactured by Malvern Instruments UK). Specifically, the Malvern Instruments Nano S uses a red (633nm) 4mW Helium-Neon laser to illuminate a standard optical quality UV curvette containing a suspension of material. The particle sizes quoted in this application are those obtained with that apparatus using the standard protocol. Particle sizes in solid products are the particle sizes inferred from the measurement of the particle size obtained by solution of the solid in water and measurement of the particle size.

The peak diameter of the water-insoluble anti-malarial drug is below 800nm. More preferably the peak diameter of the water-insoluble anti-malarial drug is below 500nm. In a particularly preferred embodiment of the invention the peak diameter of the water-insoluble anti-malarial drug is below 200nm, most preferably below 100nm.

It is believed that reduction of the particle size in the eventual nano-dispersion has significant advantages in improving the availability of the otherwise water-insoluble material. This is believed to be particularly advantageous where an improved bio-availability is sought, or, in similar applications where high local concentrations of the material are to be avoided. Moreover it is believed that nano-dispersions with a small particle size are more stable than those with a larger particle size.

In the context of the present invention, "water insoluble" as applied to the antimalarial agent means that its solubility in water is less than 10g/L.

Preferably, the water insoluble anti-malarial agent has solubility in water at ambient temperature (20 Celsius) less than 5g/L preferably of less than 1g/L, especially preferably less than 150mg/L, even more preferably less than 100mg/L. This solubility level provides the intended interpretation of what is meant by water-insoluble in the present specification.

'Artemisinin-type' drugs used in accordance with the present invention are selected from the group consisting of artemisinin (soluble at 84mg/L), artemether, arteether, dihydroartemisinin and artesunate, and mixtures thereof.

Artemisinin itself is a particularly preferred water insoluble anti-malarial drug for use in the present invention.

Compositions according to the present invention can comprise a combination of certain anti-malarial drugs. Preferred combinations include a water-insoluble 'Artemisinin-type' drug selected from artemisinin, arteether, artemether, dihydroartemisinin and artesunate, and at least one of lumefantrine, mefloquine, amodiaquine, sulfadoxine and pyrimethamine.

A further aspect of the present invention provides an aqueous dispersion of a water insoluble anti-malarial drug and a water-soluble carrier material obtainable by combining water and the water-soluble composition of the first aspect of the present invention, wherein the water-insoluble anti-malarial drug is
selected from artemisinin, arteether, artemether, dihydroartemisinin and artesunate, whether alone or in combination with another anti-malarial agent, or is selected from quinine and quinidine,
wherein said water-soluble carrier material comprises a polymer and a surfactant, the polymer being selected from polyvinylpyrrolidone (PVP), hydroxypropyl cellulose, hydroxypropylmethyl cellulose (HPMC), and mixtures thereof, the surfactant being selected from alkoxylated nonionics, phenol-ethoxylates, alkyl sulphonates, ester surfactants, cationics, and mixtures thereof,
wherein the anti-malarial drug is in nano-disperse form having a peak diameter of the nano-disperse form below 800nm, more preferably below 500nm, and especially below 200nm, most especially below 100nm. The anti-malarials discussed above being preferred in the aqueous form.

A particularly preferred aspect of the invention is that in which the particle size is below 100nm. Particles sizes as low as 25nm were obtained in the examples given below. The preferred particle distributions are such that the sizes are in the range 20-800 nm with a range of 20-200 being particularly preferred.

A further aspect of the present invention provides a process for preparing an anti-malarial composition comprising a water insoluble anti-malarial agent and a water-soluble carrier, which comprises the steps of:
a) forming an emulsion comprising:
   i) a solution of the anti-malarial agent in a water-immiscible solvent for the same, and
   ii) an aqueous solution of the carrier material, and,
b) spray-drying the emulsion to remove water and the water-immiscible solvent to obtain a substantially solvent-free nano-dispersion of the anti-malarial agent in the carrier material.

For convenience, this class of method is referred to herein as the "emulsion" method.

A further aspect of the present invention provides a process for preparing an anti-malarial composition comprising a water insoluble anti-malarial agent and a water-soluble carrier which comprises the steps of:
a) providing a mixture comprising:
   i) at least one water-miscible, non-aqueous solvent
   ii) optionally, water
   iii) a water-soluble carrier material soluble in the mixture of (i) and (ii) and
   iv) a water-insoluble anti-malarial agent which is soluble in the mixture of (i) and (ii), and,
b) spray-drying the solution to remove water and the water miscible solvent to obtain a substantially solvent-free nano-dispersion of the anti-malarial agent in the carrier material.

For convenience, this class of method is referred to herein as the "single-phase" method.

In the context of the present invention substantially solvent free means that the free solvent content of the product is less than 15%wt, preferably below 10%wt and more preferably below 5%wt.

In the context of the present invention it is essential that both the carrier material and the anti-malarial drug are essentially fully dissolved in their respective solvents prior to the drying step. It is not within the ambit of the present specification to teach the drying of slurries. For the avoidance of any doubt, it is therefore the case that the solids content of the emulsion or the mixture is such that over 90%wt, preferably over 95%, and more preferably over 98% of the soluble materials present is in solution prior to the drying step.

In relation to the methods mentioned above, the preferred anti-malarial drugs and the preferred carrier materials are as described above and as elaborated on in further detail below. Similarly the preferred physical characteristics of the material are as described above.

The 'single phase' method where both the anti-malarial agent and the carrier material are dissolved in a phase comprising at least one other non-aqueous solvent (and optional water) is preferred. This is believed to be more efficacious in obtaining a smaller particle size for the nano-disperse anti-malarial agent. The drying step simultaneously removes both the water and other solvents and is accomplished by spray drying, preferably at above ambient temperature.

The products obtainable by the process aspects of the present invention are suitable for use in the preparation of medicaments for treatment or prophylaxis of malaria.

A further aspect of the present invention provides a method for the preparation of a medicament for use in the treatment of malaria which comprises the step of preparing a composition according to the present invention.

### Detailed Description of the Invention

Various preferred features and embodiments of the present invention are described in further detail below.

### Anti-Malarial Agents

As noted above water-insoluble anti-malarial drugs for use in accordance with the invention are selected from the group consisting of artemisinin, artemether, arteether, dihydroartemisinin and artesunate, and mixtures thereof and quinine, quinidine and mixtures thereof. These can be present as the sole pharmaceutically active ingredient in compositions according to the present invention or be together with other anti-malarial drugs to provide a so-called 'combination therapy'. Suitable agents for combination therapy include lumefantrine, mefloquine, amodiaquine, sulfadoxine and pyrimethamine.

### Water-Dispersible Product Form

The present invention provides a method for obtaining a water-dispersible form of an otherwise water-insoluble material. This is prepared by forming a not wholly aqueous intermediate emulsion or solution in which both a water-soluble carrier material and the water insoluble anti-malarial are dissolved. On removal of solvents the insoluble anti-malarial material is left dispersed through the water-soluble carrier material. Suitable carrier materials are described in further detail below.

Spray-drying is particularly effective at removing both the aqueous and non-aqueous volatile components to leave the carrier and the 'payload' material behind in a powder form. The drying step is described in further detail below.

The structure of the material obtained after the drying step is not well understood. It is believed that the resulting dry materials are not encapsulates, as discrete macroscopic bodies of the water-insoluble materials are not present in the dry product. Neither are the dry materials 'dry emulsions' as little or none of the volatile solvent comprising the 'oil' phase of the emulsion remains after the drying step. On addition of water to the dry product the emulsion is not reformed, as it would be with a 'dry emulsion'. It is also believed that the compositions are not so-called solid solutions, as with the present invention the ratios of components present can be varied without loss of the benefits. Also from Xray and DSC studies, it is believed that the compositions of the invention are not solid solutions, but comprise nano-scale, phase-separated mixtures.

Preferably, the compositions produced after the drying step will comprise the anti-malarial agent and the carrier in a weight ratio of from 1:500 to 1:1 (as anti-malarial agent:carrier), 1:100 to 1:1 being preferred. Typical levels of around 10-30%wt water-insoluble anti-malarial agent and 90-70%wt carrier can be obtained by spray drying.

### 'Emulsion' Preparation Method

In this method according to the invention the solvent for the water-insoluble anti-malarial material is not miscible with water. On admixture with water it therefore can form an emulsion.

Preferably, the non-aqueous phase comprises from about 10% to about 95% v/v of the emulsion, more preferably from about 20% to about 68% v/v.

The emulsions are typically prepared under conditions which are well known to those skilled in the art, for example, by using a magnetic stirring bar, a homogeniser, or a rotational mechanical stirrer. The emulsions need not be particularly stable, provided that they do not undergo extensive phase separation prior to drying.

Homogenisation using a high-shear mixing device is a particularly preferred way to make an emulsion in which the aqueous phase is the continuous phase. It is believed that this avoidance of coarse emulsion and reduction of the droplet size of the dispersed phase of the emulsion, results in an improved dispersion of the 'payload' material in the dry product.

In a preferred method according to the invention a water-continuous emulsion is prepared with an average dispersed-phase droplet size (using the Malvern peak intensity) of between 500nm and 5000nm. We have found that an 'Ultra-Turrux' T25 type laboratory homogenizer (or equivalent) gives a suitable emulsion when operated for more than a minute at above 10,000 rpm.

There is a directional relation between the emulsion droplet size and the size of the particles of the 'payload' material, which can be detected after dispersion of the materials of the invention in aqueous solution. We have determined that an increase in the speed of homogenization for precursor emulsions can decrease final particle size after re-dissolution.

It is believed that the re-dissolved particle size can be reduced by nearly one half when the homogenization speed increased from 13,500 rpm to 21,500 rpm. The homogenization time is also believed to play a role in controlling re-dissolved particle size. The particle size again decreases with increase in the homogenization time, and the particle size distribution become broader at the same time.

Sonication is also a particularly preferred way of reducing the droplet size for emulsion systems. We have found that a Hert Systems Sonicator XL operated at level 10 for two minutes is suitable.

It is believed that ratios of components which decrease the relative concentration of the anti-malarial to the solvents and/or the carrier give a smaller particle size.

### 'Single Phase' Preparation Method

In an alternative method according to the present invention both the carrier and the anti-malarial agent are soluble in a non-aqueous solvent or a mixture of such a solvent with water. Both here and elsewhere in the specification the non-aqueous solvent can be a mixture of non-aqueous solvents.

In this case the feedstock of the drying step can be a single phase material in which both the water-soluble carrier and the water-insoluble anti-malarial agent are dissolved. It is also possible for this feedstock to be an emulsion, provided that both the carrier and the agent are dissolved in the same phase.

The 'single-phase' method is generally believed to give a better nano-dispersion with a smaller particle size than the emulsion method.

It is believed that ratios of components which decrease the relative concentration of the anti-malarial to the solvents and/or the carrier give a smaller particle size.

### Drying

Spray drying is well known to those versed in the art. In the case of the present invention some care must be taken due to the presence of a volatile non-aqueous solvent in the emulsion being dried. In order to reduce the risk of explosion when a flammable solvent is being used, an inert gas, for example nitrogen, can be employed as the drying medium in a so-called closed spray-drying system. The solvent can be recovered and re-used.

We have found that the 'Buchi' B-290 type laboratory spray drying apparatus is suitable.

It is preferable that the spray-drying temperature should be at or above 100 Celsius, preferably above 120 Celsius and most preferably above 140 Celsius. Elevated drying temperatures have been found to give smaller particles in the re-dissolved nano-disperse material.

### Carrier Material

The carrier material is water soluble, which includes the formation of structured aqueous phases as well as true ionic solution of molecularly mono-disperse species. The carrier material comprises a polymer and a surfactant. In accordance with the invention, the polymer is selected from polyvinylpyrrolidone (PVP), hydroxypropyl cellulose, hydroxypropyl methylcellulose (HPMC), and mixtures thereof, whilst the surfactant is selected from alkoxylated nonionics, phenolethoxylates, alkyl sulphonates, ester surfactants, cationics, and mixtures thereof.

The carrier material may also comprise, in addition to the polymer and surfactant, an inorganic material.

Furthermore, other non-polymeric, organic, water-soluble materials such as sugars may also be used, in addition to the polymer and surfactant, as the carrier.

Mixtures of surfactants may be used. In such mixtures there may be individual components which are liquid, provided that the carrier material overall, is a solid.

Alkoxylated nonionic's (especially the PEG/PPG Pluronic™ materials), phenol-ethoxylates (especially TRITON™ materials), alkyl sulphonates (especially SDS), ester surfactants (preferably sorbitan esters of the Span™ and Tween™ types) and cationics (especially cetyltrimethylammonium bromide - CTAB) are surfactant carrier materials used in accordance with the present invention.

### Preferred, additional inorganic carrier materials

As noted above, the carrier material can further comprise, in addition to the polymer and surfactant, a water-soluble inorganic material which is neither a surfactant nor a polymer. Simple inorganic salts have been found suitable, in admixture with the polymeric and surfactant carrier materials described above. Suitable salts include carbonate, bicarbonates, halides, sulphates, nitrates and acetates, particularly soluble salts of sodium, potassium and magnesium. Preferred materials include sodium carbonate, sodium bicarbonate and sodium sulphate. These materials have the advantage that they are cheap and physiologically acceptable. They are also relatively inert as well as compatible with many materials found in pharmaceutical products.

### Preferred, additional organic carrier materials

As noted above, the carrier material can further comprise, in addition to the polymer and surfactant, a water-soluble small organic material which is neither a surfactant, nor a polymer nor an inorganic carrier material. Simple organic sugars have been found to be suitable, in admixture with the polymeric and surfactant carrier materials described above. Suitable small organic materials include mannitol, polydextrose, xylitol and inulin etc.

### Non-aqueous solvent

The compositions of the invention comprise a volatile, second non-aqueous solvent. This may either be miscible with the other solvents in pre-mix before drying or, together with those solvents may form an emulsion.

In one alternative form of the invention a single, nonaqueous solvent is employed in which can form a single phase with water in the presence of the anti-malarial agent, and the carrier. Preferred solvents for these embodiments are polar, protic or aprotic solvents. Generally preferred solvents have a dipole moment greater than 1 and a dielectric constant greater than 4.5.

Particularly preferred solvents are selected from the group consisting of haloforms (preferably dichloromethane, chloroform), lower (C1-C10) alcohols (preferably methanol, ethanol, isopropanol, isobutanol), organic acids (preferably formic acid, acetic acid), amides (preferably formamide, N,N-dimethylformamide), nitriles (preferably aceto-nitrile), esters (preferably ethyl acetate) aldehydes and ketones (preferably methyl ethyl ketone, acetone), and other water miscible species comprising hetroatom bond with a suitably large dipole (preferably tetrahydrofuran, dialkylsulphoxide).

Haloforms, lower alcohols, ketones and dialkylsulphoxides are the most preferred solvents.

In another alternative form of the invention the non-aqueous solvent is not miscible with water and forms an emulsion.

The non-aqueous phase of the emulsion is preferably selected from one or more from the following group of volatile organic solvents:
- alkanes, preferably heptane, n-hexane, isooctane, dodecane, decane;
- cyclic hydrocarbons, preferably toluene, xylene, cyclohexane;
- halogenated alkanes , preferably dichloromethane, dichoroethane, trichloromethane (chloroform), fluorotrichloromethane and tetrachloroethane;
- esters preferably ethyl acetate;
- ketones preferably 2-butanone;
- ethers preferably diethyl ether;
- volatile cyclic silicones preferably either linear or cyclomethicones containing from 4 to 6 silicon units. Suitable examples include DC245 and DC345, both of which are available from Dow Corning Inc.

Preferred solvents include dichloromethane, chloroform, ethanol, acetone and dimethyl sulphoxide.

Preferred non-aqueous solvents, whether miscible or not have a boiling point of less than 150 Celsius and, more preferably, have a boiling point of less than 100 Celsius, so as to facilitate drying, particularly spray-drying under practical conditions and without use of specialised equipment. Preferably they are non-flammable, or have a flash point above the temperatures encountered in the method of the invention.

Preferably, the non-aqueous solvent comprises from about 10 % to about 95% v/v of any emulsion formed, more preferably from about 20% to about 80 % v/v. In the single phase method the level of solvent is preferably 20-100%v/v.

Particularly preferred solvents are alcohols, particularly ethanol and halogenated solvents, more preferably chlorine-containing solvents, most preferably solvents selected from (di- or tri- chloromethane).

### Optional Cosurfactant

In addition to the non-aqueous solvent an optional co-surfactant may be employed in the composition prior to the drying step. We have determined that the addition of a relatively small quantity of a volatile cosurfactant reduced the particle diameter of the material produced. This can have a significant impact on particle volume. For example, reduction from 297nm to 252nm corresponds to a particle size reduction of approximately 40%. Thus, the addition of a small quantity of co-surfactant offers a simple and inexpensive method for reducing the particle size of materials according to the present invention without changing the final product formulation.

Preferred co-surfactants are short chain alcohols or amine with a boiling point of <220°C.

Preferred co-surfactants are linear alcohols. Preferred co-surfactants are primary alcohols and amines. Particularly preferred co-surfactants are selected from the group consisting of the 3-6 carbon alcohols. Suitable alcohol co-surfactants include n-propanol, n-butanol, n-pentanol, n-hexanol, hexylamine and mixtures thereof.

Preferably the co-surfactant is present in a quantity (by volume) less than the solvent preferably the volume ratio between the solvent and the co-surfactant falls in the range 100:40 to 100:2, more preferably 100:30 to 100:5.

### Preferred Spray-Drying Feedstocks

Typical spray drying feedstocks comprise:
a) a surfactant,
b) at least one lower alcohol,
c) more than 0.1% of at least one water-insoluble anti-malarial agent dissolved in the feedstock,
d) a polymer, and,
e) optional water

Preferred spray-drying feedstocks comprise:
a) at least one non-aqueous solvent selected from dichloromethane, chloroform, ethanol, acetone, and mixtures thereof,
b) a surfactant selected from PEG co-polymer nonionic's (especially the PEG/PPG Pluronic™ materials), alkyl sulphonates (especially SDS), ester surfactants (preferably sorbitan esters of the Span™ and Tween™ types) and cationics (especially cetyltrimethylammonium bromide - CTAB) and mixtures thereof,
c) more than 0.1% of at least one water-insoluble anti-malarial agent selected from artemisinin, arteether, artemether, dihydroartemisinin and artesunate, whether alone or in combination with another antimalarial agent, or is selected from quinine and quinidine,
d) a polymer selected from polyvinylpyrrolidone (PVP), hydroxypropyl cellulose and hydroxypropyl-methyl cellulose (HPMC), and mixtures thereof, and
e) optionally water.

The drying feed-stocks used in the present invention are either emulsions or solutions which preferably do not contain solid matter and in particular preferably do not contain any undissolved anti-malarial agent.

It is particularly preferable that the level of the anti-malarial agent in the composition should be such that the loading in the dried composition is below 40%wt, and more preferably below 30%wt. Such compositions have the advantages of a small particle size and high effectiveness as discussed above.

### Water-Dispersed Form

On admixture of the water-soluble carrier material with water, the carrier dissolves and the water-insoluble anti-malarial agent is dispersed through the water in sufficiently fine form that it behaves like a soluble material in many respects. The particle size of the water-insoluble materials in the dry product is preferably such that, on solution in water the water-insoluble materials have a particle size of less than 1 micron as determined by the Malvern method described herein. It is believed that there is no significant reduction of particle size for the anti-malarial agent on dispersion of the solid form in water.

By applying the present invention significant levels of 'water-insoluble' materials can be brought into a state which is largely equivalent to true solution. When the dry product is dissolved in water it is possible to achieve optically clear solutions comprising more than 0.1%, preferably more than 0,5% and more preferably more than 1% of the water-insoluble material.

It is envisaged that the solution form will be a form suitable for administration to a patient either 'as is' or following further dilution. In the alternative, the solution form of embodiments of the invention may be combined with other active materials to yield a medicament suitable for use in combination therapy.

In order that the present invention may be further understood and carried forth into practice it is further described below with reference to non-limiting examples.

### Examples:

For each sample (unless stated otherwise), about 10 mg powder was re-dispersed into 10 ml distilled water at room temperature (21.5 °C) to give a 1mg/ml nano-dispersion for particle size measurements.

A method of particle sizing for the dispersed products of the present invention used in the following examples employs a dynamic light scattering instrument (Nano S, manufactured by Malvern Instruments UK). Specifically, the Malvern Instruments Nano S uses a red (633nm) 4mW Helium-Neon laser to illuminate a standard optical quality UV curvette containing a suspension of material.

For convenience the results in the first twelve examples (some of which are comparative) are summarised in the table below:

| **Ex.** | **Solvent** | **Carrier** | **Method** | **Particle size** |
|---|---|---|---|---|
| 1 | Ethanol, Water | PEG, HPMC | Single phase (100C spray) | 695 |
| 2 | Ethanol, Water | Pluronic, HPMC | Single phase (100C spray) | 770 |
| 3 | Ethanol, Water | Pluronic, PVP | Single phase (100C spray) | 705 |
| 4 | Ethanol, Water | Cyclodextrin, HPMC | Single phase (100C spray) | 667 |
| 5 | Ethanol, Water | PEG, HPMC | Single phase (150C spray) | 56 |
| 6 | Ethanol, Water | Pluronic, HPMC | Single phase (150C spray) | 42 |
| 7 | Ethanol, Water | Lipoid, HPMC | Single phase (150C spray) | 211 |
| 8 | Ethanol, Water | Pluronic, HPMC, CTAB | Single phase (150C spray) | 25 |
| 9 | Ethanol, Water | Pluronic, HPMC, SDS | Single phase (150C spray) | 39 |
| 10 | Ethanol, Water | Pluronic, HPMC, CTAB, SDS | Single phase (150C spray) | 331 |
| 11 | Ethanol | Pluronic, Span, Klucel | Single phase (150C spray) | 180-182 |
| 12 | Ethanol | Pluronic, Span, Klucel | Single phase (150C spray) | 183-207 |

### Comparative Example 1:

0.10 g artemisinin (99 %, supplied by Hunan Keyuan Biology Product Co. Ltd, China) and 0.05 g polyethylene glycol (PEG, Mw 3,000, Fluka) were both dissolved into 50 ml ethanol. 0.85 g Hydroxypropyl methylcellulose (HPMC, 5cps, Aldrich) was added into the ethanol solution with intensive stirring using a magnetic bar to form a uniform HPMC/EtOH suspension. 50 ml distilled water was then added into the suspension, and a clear solution was obtained. The solution was then spray dried (BUCHI Mini-290) at 100 °C with liquid feed rate 2.5 ml/min. A white powder with 10 wt% (theoretical) artemisinin was obtained and collected in a sample vial for further characterization.

The particle size of the redispersed material (d,nm) was 695+/-15.

### Example 2:

0.10 g artemisinin and 0.10 g pluronic F-68 (BASF, USP) were both dissolved into 50 ml ethanol. 0.80 g HPMC was added into the ethanol solution with intensive stirring using a magnetic bar to form a uniform HPMC/EtOH suspension. 50 ml distilled water was then added into the suspension, and a clear solution was obtained. The solution was then spray dried at 100 °C with liquid feed rate 2.5 ml/min. A white powder with 10 wt% (theoretical) artemisinin was obtained and collected in a sample vial for further characterization.

The particle size of the redispersed material (d,nm) was 770+/-27.

### Example 3:

0.10 g artemisinin and 0.10 g pluronic F-68 were both dissolved into 50 ml ethanol. 0.80 g polyvinylpyrrolidone (PVP k30, Aldrich) was added into the ethanol solution following 50 ml distilled water, and a clear solution was obtained. The solution was then spray dried at 100 °C with liquid feed rate 2.5 ml/min. A white powder with 10 wt% (theoretical) artemisinin was obtained and collected in a sample vial for further characterization.

The particle size of the redispersed material (d,nm) was 705+/-64.

### Comparative Example 4:

0.20 g artemisinin was dissolved into 50 ml ethanol. 0.40 g HPMC and 0.40 g beta-cyclodextrin (Aldrich) were both dispersed into the ethanol solution with stirring using a magnetic bar to form a uniform HPMC/EtOH suspension. 50 ml distilled water was then added into the suspension, and a clear solution was obtained. The solution was then spray dried at 100 °C with liquid feed rate 2.5 ml/min. A white powder with 20 wt% (theoretical) artemisinin was obtained and collected in a sample vial for further characterization,

The particle size of the redispersed material (d,nm) was 667+/-35.

### Comparative Example 5:

0.10 g Artemisinin and 0.05 g PEG (Mw 6,000, Fluka) were both dissolved into 50 ml ethanol. 0.85 g HPMC (5 cps, Aldrich) was added into the ethanol solution with intensive stirring using a magnetic bar in order to form a uniform HPMC/EtOH suspension. 50 ml Distilled water was then added into the suspension, and a clear solution was obtained. The solution was then spray dried at 150 °C with liquid feed rate 2.5 ml/min. A white powder with 10 wt% (theoretical) artemisinin was obtained and collected in a sample vial for further characterization.

The particle size of the redispersed material (d,nm) was 56+/-3.

### Example 6:

0.10 g Artemisinin and 0.10 g Pluronic F-127 (Aldrich) were both dissolved into 50 ml ethanol. 0.80 g HPMC (5 cps, Aldrich) was added into the ethanol solution with intensive stirring using a magnetic bar to form a uniform HPMC/EtOH suspension. 50 ml distilled water was then added into the suspension, and a clear solution was obtained. The solution was then spray dried at 150 °C with liquid feed rate 2.5 ml/min. A white powder with 10 wt% (theoretical) artemisinin was obtained and collected in a sample vial for further characterization.

The particle size of the redispersed material (d,nm) was 42+/-14.

### Example 7:

0.10 g Artemisinin and 0.10 g lipoid S75 (Lipoid GmbH) were both dissolved into 50 ml ethanol. 0.80 g HPMC (5 cps, Aldrich) was added into the ethanol solution with intensive stirring using a magnetic bar to form a uniform HPMC/EtOH suspension. 50 ml Distilled water was then added into the suspension, and a clear solution was obtained. The solution was then spray dried at 150 °C with liquid feed rate 2.5 ml/min. A white powder with 10 wt% (theoretical) artemisinin was obtained and collected in a sample vial for further characterization.

The particle size of the redispersed material (d,nm) was 211+/-5.

### Example 8:

0.10 g Artemisinin and 0.09 g Pluronic F-127 were both dissolved into 50 ml ethanol. 0.80 g HPMC (5 cps, Aldrich) and 0.01 g Cetrimide (Cetyltrimethylammonium Bromide, Aldrich) were added into the ethanol solution with intensive stirring using a magnetic bar to form a uniform HPMC/EtOH suspension. 50 ml Distilled water was then added into the suspension, and a clear solution was obtained. The solution was then spray dried at 150 °C with liquid feed rate 2.5 ml/min. A white powder with 10 wt% (theoretical) artemisinin was obtained and collected in a sample vial for further characterization.

The particle size of the redispersed material (d,nm) was 25+/-2.

### Example 9:

0.10 g Artemisinin and 0.09 g Pluronic F-127 were both dissolved into 50 ml ethanol. 0.80 g HPMC (5 cps, Aldrich) and 0.01 g SDS (Aldrich) were added into the ethanol solution with intensive stirring using a magnetic bar to form a uniform HPMC/EtOH suspension. 50 ml Distilled water was then added into the suspension, and a clear solution was obtained. The solution was then spray dried at 150°C with liquid feed rate 2.5 ml/min. A white powder with 10 wt% (theoretical) artemisinin was obtained and collected in a sample vial for further characterization.

The particle size of the redispersed material (d,nm) was 39+/-9.

### Example 10:

0.10 g Artemisinin and 0.09 g Pluronic F-127 were both dissolved into 50 ml ethanol. 0.80 g HPMC (5 cps, Aldrich), 0.005 g Cetrimide, and 0.005 g SDS were all added into the ethanol solution with intensive stirring using a magnetic bar to form a uniform HPMC/EtOH suspension. 50 ml Distilled water was then added into the suspension, and a clear solution was obtained. The solution was then spray dried at 150 °C with liquid feed rate 2.5 ml/min. A white powder with 10 wt% (theoretical) artemisinin was obtained and collected in a sample vial for further characterization.

The particle size of the redispersed material (d,nm) was 331+/-9.

### Example 11:

0.20 g Artemisinin, 0.05 g Pluronic F68, 0.05 g Span 80 (Aldrich), and 0.70 g Klucel EF (Hydroxypropyl cellulose, Mw 80,000, Hercules Ltd) were all dissolved into 70 ml ethanol. The solution was then spray dried at 150 °C with liquid feed rate 2.5 ml/min. A white powder with 20 wt% (theoretical) artemisinin was obtained and collected in a sample vial for further characterization.

The particle size of the redispersed material (d,nm) was measured at 182+/-4 when dispersed at lmg/ml and 180+/-2 when dispersed at 2 mg/ml.

### Example 12:

0.30 g Artemisinin, 0.08 g Pluronic F68, 0.07 g Span 80 (Aldrich), and 0.55 g Klucel EF were all dissolved into 70 ml ethanol. The solution was then spray dried at 150 °C with liquid feed rate 2.5 ml/min. A white powder with 30 wt% (theoretical) artemisinin was obtained and collected in a sample vial for further characterization.

The particle size of the redispersed material (d,nm) was measured at 183+/-2 when dispersed at 1mg/ml and 207+/-13 when dispersed at 2 mg/ml.

### Comparative Example 13:

0.1015 g Artemisinin was prepared with EtOH in 100 ml volumetric flask as a standard solution. Six aliquots equivalent to 0, 0.50, 1.00, 1.50, 2.00, and 3.00 ml of standard solution were pipetted into six 50 ml volumetric flasks, respectively. The solutions were then diluted up to 5.00 ml with ethanol, using a pipette, and mixed with 20 ml of 0.2 wt% NaOH solutions, respectively. These mixtures were then warmed in a water bath at 50 °C for 40 min to obtain a new chemical named Q292, which has UV absorption at 292 nm. After being cooled to room temperature in water, these mixtures were acidified by adding 0.08 M acetic acid to make up the volumes, and a new chemical named Q260 with UV absorption at 260 nm was obtained. This follows the method of G Qian, Y Yang, Q Ren, (*Determination of Artemisinin in* Artemisia annua L. by Reversed Phase HPLC, J. Liquid Chromatography & Related Technologies, 28, 2005). The solutions were then analysed by UV to obtain a calibration curve. An example with a theoretical level of 15% was analysed with the method to identify the artemisinin content in solid powder. The analysis found that there was 13.88 wt% artemisinin in the powder.

### Example 14-17:

A solution of the excipients and active (for example 14), was made by dissolving 0.1g of Quinine, 0.62g Klucel (Hercules incorporated), 0.03g of Span 80 (Sigma Aldrich), 0.2g of Tween 80 (Croda) and 0.05g of Cremophor ELP (No. 35)(BASF Chem. Trade), (see table below) in 80ml of ethanol and 40ml of water. The solution placed was on a magnetic stirrer for 5-10 minutes to ensure all of the excipients and active were completely dispersed.

The solution was spray dried on a Buchi B290 Mini spray-drier with a pump rate of 10% and inlet temperature of 140°C. In all cases a white powder was obtained.
The total powder content in each formulation was Ig dissolved in 50ml of ethanol and 20ml deionised water. Extra ethanol was added if the solution was cloudy, until it turned clear and all the excipients were dissolved.

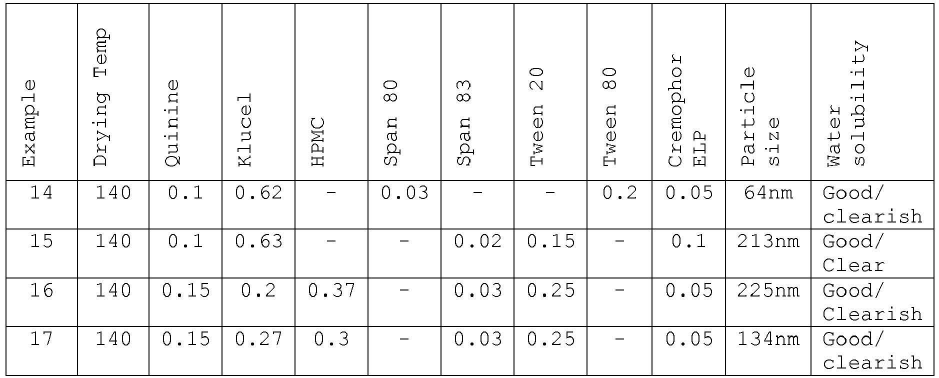

Dissolution tests were carried out on examples 14 and 15. The dissolution vessel was filled with a 1L of pre-warmed deionised water placed in a pre-warmed water bath set at 37°C. Each powder was added to the vessel containing the IL of water with constant stirring as follows: example 14 2000mg (equivalent to 200mg quinine) powder, and example 16 approximately 2707mg (equivalent to 271mg quinine).

Aliquots of 2.5 -5ml were collected using a pipette at set time intervals of 1, 5, 10 and 20minutes. In each test a further aliquot was collected at 30 minutes because not all the powder dissolved and the speed of the overhead stirring paddle was increased to 140rpm to speed up the dissolution. One final aliquot was then collect to be used as the equilibrium concentration at the end of the test.

The tables below show the % Dissolution Vs Time:

### Example 14

| **Time (mins)** | **% Dissolved** | **Time (mins)** | **% Dissolution** |
|---|---|---|---|
| 1 | 60.8 | 1 | 48.6 |
| 5 | 81.7 | 5 | 65.4 |
| 10 | 84.7 | 10 | 67.7 |
| 20 | 86.8 | 20 | 69.4 |
| 30 | 87.3 | 30 | 69.8 |
| 115 | 124.3 | 115 | 99.5 |

### Example 16:

| **Time (mins)** | **% Dissolved** | **Time (mins)** | **% Dissolution** |
|---|---|---|---|
| 1 | 59.9 | 1 | 55.8 |
| 5 | 83.9 | 5 | 78.2 |
| 10 | 94 | 10 | 87.5 |
| 20 | 106.9 | 20 | 99.5 |
| 30 | 110.2 | 30 | 102.6 |

### Example 18a-f:

### 18a

0.2 g artemisinin and 0.05 g Span 80 were both dissolved into 20 ml Chloroform. 0.05 g Pluronic F68, 0.65 g Klucel EF and 0.05 g sodium alginate were all dissolved into 80 ml distilled water. The oil phase was added dropwise into the aqueous phase with overhead stirring at 600 rpm for 2 min. The coarse emulsion was further treated with a homogenizer (Yellowline DI25 Basic) at 13,500 rpm for 5 min. The fine emulsion was then spray dried at 150 °C with a Buchi Mini spray dryer B-290, and the emulsion droplet size was measured with a Malvern Nano-S. 10 mg dry powder was then dispersed into 10 ml distilled water and the nanoparticle size was measured with the Malvern Nano-S.

### 18b

Using the same formulation as example 18a, the coarse emulsion was further treated with a homogenizer at 20,500 rpm for 10 min. The fine emulsion was then spray dried at 150 °C with a Buchi Mini spray dryer B-290, and the emulsion droplet size was measured with the Malvern Nano-S. 10 mg dry powder was then dispersed into 10 ml distilled water and the nanoparticle size was measured with the Malvern Nano-S.

### 18c

Using the same formulation as example 18a, the coarse emulsion was further treated with a homogenizer at 24,000 rpm for 5 min. The fine emulsion was then spray dried at 150 °C with a Buchi Mini spray dryer B-290, and the emulsion droplet size was measured with the Malvern Nano-S. 10 mg dry powder was then dispersed into 10 ml distilled water and the nanoparticle size was measured with the Malvern Nano-S.

### 18d

Using the same formulation as example 18a, the coarse emulsion was further treated with a homogenizer at 24,000 rpm for 10 min. The fine emulsion was then spray dried at 150 °C with a Buchi Mini spray dryer B-290, and the emulsion droplet size was measured with the Malvern Nano-S. 10 mg dry powder was then dispersed into 10ml distilled water and the nanoparticle size was measured with the Malvern Nano-S.

### 18e

Using the same formulation as example 18a, the coarse emulsion was further treated with an ultrasonic probe (Sonicator®, Ultrasonic Processor XL) for 1 min. The fine emulsion was then spray dried at 150 °C with a Buchi Mini spray dryer B-290, and the emulsion droplet size was measured with the Malvern Nano-S. 10 mg dry powder was then dispersed into 10 ml distilled water and the nanoparticle size was measured with the Malvern Nano-S.

### 18f

Using the same formulation as example 18a, the coarse emulsion was further treated with an ultrasonic probe at for 3 min. The fine emulsion was then spray dried at 150 °C with a Buchi Mini spray dryer B-290, and the emulsion droplet size was measured with the Malvern Nano-S. 10 mg dry powder was then dispersed into 10 ml distilled water and the nanoparticle size was measured with the Malvern Nano-S.

Further concerning these experiments is given in the following table

| **Examples** | **18a** | **18b** | **18c** | **18d** | **18e** | **18f** |
|---|---|---|---|---|---|---|
| Emulsion Droplet Size, nm | 3217 | 2928 | 2454 | 2293 | 1730 | 900 |
| Artemisinin Nanoparticle size, nm | 636 | 467 | 311 | 273 | 146 | 101 |

## Claims

1. A spray-dried, water-soluble composition comprising at least one water-insoluble anti-malarial drug and a water-soluble carrier material,
wherein the water-insoluble anti-malarial drug is selected from artemisinin, arteether, artemether, dihydroartemisinin and artesunate, whether alone or in combination with another anti-malarial agent, or is selected from quinine and quinidine,
wherein said water-soluble carrier material comprises a polymer and a surfactant,
the polymer being selected from polyvinylpyrrolidone (PVP), hydroxypropyl cellulose, hydroxypropyl methyl cellulose (HPMC), and mixtures thereof, and the surfactant being selected from alkoxylated nonionics, phenol-ethoxylates, alkyl sulphonates, ester surfactants, cationics, and mixtures thereof, and wherein the water-insoluble anti-malarial drug is dispersed through the water-soluble carrier material in nano-disperse form having a peak diameter of the nano-disperse form within the composition, as measured as described herein, of 20-800 nm.

2. A composition according to claim 1 wherein the anti-malarial drug has a water solubility of less than 5 g/L.

3. A composition according to claim 1 or claim 2, wherein the anti-malarial drug is selected from the group comprising artemisinin, artemether, arteether, dihydroartemisinin and mixtures thereof.

4. An aqueous dispersion of a water-insoluble anti-malarial drug and a water-soluble carrier material obtainable by combining water and the water-soluble composition of any one of claims 1-3,
wherein the water-insoluble anti-malarial drug is selected from artemisinin, arteether, artemether, dihydroartemisinin and artesunate, whether alone or in combination with another anti-malarial agent, or is selected from quinine and quinidine,
wherein said water-soluble carrier material comprises a polymer and a surfactant,
the polymer being selected from polyvinylpyrrolidone (PVP), hydroxypropyl cellulose, hydroxypropyl methyl cellulose (HPMC), and mixtures thereof, and the surfactant being selected from alkoxylated nonionics, phenol-ethoxylates, alkyl sulphonates, ester surfactants, cationics, and mixtures thereof,
wherein the anti-malarial drug is in nano-disperse form having a peak particle diameter measured as described herein of 20-800 nm.

5. A process for preparing a water-soluble composition according to any one of claims 1-3 which comprises the steps of:
a) providing an emulsion comprising:
i) a solution of the water-insoluble anti-malarial drug in a water-immiscible solvent for the same, and
ii) an aqueous solution of the water-soluble carrier material, and,
b) spray-drying the emulsion to remove water and the water-immiscible solvent to obtain a substantially solvent-free nano-dispersion of the anti-malarial drug in the carrier material.

6. A process for preparing a water-soluble composition according to any one of claims 1-3 which comprises the steps of:
a) providing a mixture comprising:
i) at least one water-miscible, non-aqueous solvent
ii) optionally, water
iii) a water-soluble carrier material soluble in the mixture of (i) and (ii) and
iv) a water-insoluble anti-malarial drug which is soluble in the mixture of (i) and (ii), and,
b) spray-drying the solution to remove water and the water-miscible solvent to obtain a substantially solvent-free nano-dispersion of the anti-malarial drug in the carrier material.

7. A process according to claim 5 or claim 6 wherein the spray drying process is conducted at a temperature of above 120 Celsius.

8. A process according to any one of claims 5-7 wherein the non-aqueous solvent includes at least one of dichloromethane, chloroform, ethanol, acetone and dimethyl sulphoxide.

9. A process for the preparation of a medicament for use in the treatment or prophylaxis of malaria which comprises the step of preparing a water-soluble composition according to any one of claims 1-3.

## Patentansprüche

1. Sprühgetrocknete wasserlösliche Zusammensetzung, umfassend mindestens einen wasserunlöslichen Anti-Malaria-Arzneistoff und ein wasserlösliches Trägermaterial,
wobei der wasserunlösliche Anti-Malaria-Arzneistoff ausgewählt ist aus Artemisinin, Arteether, Artemether, Dihydroartemisinin und Artesunat, ob allein oder in Kombination mit anderem Anti-Malaria-Mittel, oder ausgewählt aus Chinin und Chinidin,
wobei das wasserlösliche Trägermaterial ein Polymer und ein Tensid umfasst, wobei das Polymer ausgewählt ist aus Polyvinylpyrrolidon (PVP), Hydroxypropylcellulose, Hydroxypropylmethylcellulose (HPMC) und Mischungen davon, und
das Tensid ausgewählt ist aus alkoxylierten nichtionischen Stoffen, Phenolethoxylaten, Alkylsulfonaten, Ester-Tensiden, kationischen Stoffen und Mischungen davon, und
wobei der wasserunlösliche Anti-Malaria-Arzneistoff durch das wasserlösliche Trägermaterial in nanodisperser Form dispergiert wird, das einen Peak-Durchmesser der nanodispersen Form innerhalb der Zusammensetzung von 20 bis 800 nm aufweist, der wie hierin beschrieben gemessen wird.

2. Zusammensetzung nach Anspruch 1, wobei der Anti-Malaria-Arzneistoff eine Wasserlöslichkeit von weniger als 5 g/l aufweist.

3. Zusammensetzung nach Anspruch 1 oder Anspruch 2, wobei der Anti-Malaria-Arzneistoff ausgewählt ist aus der Gruppe umfassend Artemisinin, Artemether, Arteether, Dihydroartemisinin und Mischungen davon.

4. Wässrige Dispersion eines wasserunlöslichen Anti-Malaria-Arzneistoffs und eines wasserlöslichen Trägermaterials, das erhalten werden kann durch Vereinen von Wasser und der wasserlöslichen Zusammensetzung nach einem der Ansprüche 1 bis 3,
wobei der wasserunlösliche Anti-Malaria-Arzneistoff ausgewählt wird aus Artemisinin, Arteether, Artemether, Dihydroartemisinin und Artesunat, ob allein oder in Kombination mit anderem Anti-Malaria-Mittel, oder ausgewählt wird aus Chinin und Chinidin,
wobei das wasserlösliche Trägermaterial ein Polymer und ein Tensid umfasst,
wobei das Polymer ausgewählt ist aus Polyvinylpyrrolidon (PVP), Hydroxypropylcellulose, Hydroxypropylmethylcellulose (HPMC) und Mischungen davon, und
das Tensid ausgewählt ist aus alkoxylierten nichtionischen Stoffen, Phenolethoxylaten, Alkylsulfonaten, Ester-Tensiden, kationischen Stoffen und Mischungen davon, und
wobei der wasserunlösliche Anti-Malaria-Arzneistoff in nanodisperser Form vorliegt und einen Peak-Partikeldurchmesser von 20 bis 800 nm aufweist, der wie hierin beschrieben gemessen wird.

5. Verfahren zum Herstellen einer wasserlöslichen Zusammensetzung nach einem der Ansprüche 1 bis 3, das die Schritte umfasst:
a) Bereitstellen einer Dispersion, umfassend:
i) eine Lösung des wasserunlöslichen Anti-Malaria-Arzneistoffs in einem mit Wasser nichtmischbaren Lösemittel desselben, und
ii) eine wässrige Lösung des wasserlöslichen Trägermaterials, und
b) Sprühtrocknen der Emulsion, um Wasser und das mit Wasser nicht mischbare Lösemittel zu entfernen, um eine weitgehend lösemittelfreie Nano-Dispersion des Anti-Malaria- Arzneistoffs in dem Trägermaterial zu erhalten.

6. Verfahren zum Herstellen einer wasserlöslichen Zusammensetzung nach einem der Ansprüche 1 bis 3, das die Schritte umfasst:
a) Bereitstellen einer Mischung, umfassend:
i mindestens ein mit Wasser mischbares, nichtwässriges Lösemittel
ii) wahlweise Wasser
iii) ein wasserlösliches Trägermaterial, das in der Mischung von (i) und (ii) löslich ist, und
iv) einen wasserunlöslichen Anti-Malaria-Arzneistoff, der in der Mischung von (i) und (ii) löslich ist, und
b) Sprühtrocknen der Lösung, um Wasser und das mit Wasser mischbare Lösemittel zu entfernen, um eine weitgehend lösemittelfreie Nano-Dispersion des Anti-Malaria-Arzneistoffs in dem Trägermaterial zu erhalten.

7. Verfahren nach Anspruch 5 oder Anspruch 6, wobei das Verfahren des Sprühtrocknens bei einer Temperatur oberhalb von 120 °C ausgeführt wird.

8. Verfahren nach einem der Ansprüche 5 bis 7, wobei das nichtwässrige Lösemittel mindestens eines der Folgenden einschließt: Dichlormethan, Chloroform, Ethanol, Aceton und Dimethylsulfoxid.

9. Verfahren für die Herstellung eines Medikaments zur Verwendung in der Behandlung oder Prophylaxe von Malaria, das den Schritt des Herstellens einer wasserlöslichen Zusammensetzung nach einem der Ansprüche 1 bis 3 umfasst.

## Revendications

1. Composition soluble à l'eau séchée par atomisation comprenant au moins un médicament antipaludique non soluble à l'eau et un matériau de support soluble à l'eau ;
dans laquelle le médicament antipaludique non soluble à l'eau est sélectionné parmi l'artémisinine, l'artééther, l'artéméther, la dihydroartémisinine et l'artésunate, qu'il soit seul ou en combinaison avec un autre agent antipaludique, ou est sélectionné parmi la quinine et la quinidine ; dans laquelle :
ledit matériau de support soluble à l'eau comprend un polymère et un agent tensioactif ;
le polymère étant sélectionné parmi le polyvinylpyrrolidone (PVP), l'hydroxypropylcellulose, l'hydroxypropyle méthyl cellulose (HPMC) et des mélanges afférents ; et
l'agent tensioactif étant sélectionné parmi les agents non ioniques alcoxylatés, les phénoléthoxylates, les alkylsulfonates, les agents tensioactifs à base d'ester, les agents cationiques et des mélanges afférents ; et
dans laquelle le médicament antipaludique non soluble à l'eau est dispersé au sein du matériau de support soluble à l'eau sous une forme nano-dispersée qui présente un diamètre de crête de la forme nano-dispersée à l'intérieur de la composition, tel que mesuré comme il est décrit ici, de 20 nm à 800 nm.

2. Composition selon la revendication 1, dans laquelle le médicament antipaludique présente une solubilité à l'eau inférieure à 5 g/L.

3. Composition selon la revendication 1 ou la revendication 2, dans laquelle le médicament antipaludique est sélectionné parmi le groupe qui comprend l'artémisinine, l'artéméther, l'artééther, la dihydroartémisinine et des mélanges afférents.

4. Dispersion aqueuse d'un médicament antipaludique non soluble à l'eau et d'un matériau de support soluble à l'eau qui peut être obtenue en combinant de l'eau et la composition soluble à l'eau selon l'une quelconque des revendications 1 à 3 ; dans laquelle :
le médicament antipaludique non soluble à l'eau est sélectionné parmi l'artémisinine, l'artééther, l'artéméther, la dihydroartémisinine et l'artésunate, qu'il soit seul ou en combinaison avec un autre agent antipaludique, ou est sélectionné parmi la quinine et la quinidine ; dans laquelle :
ledit matériau de support soluble à l'eau comprend un polymère et un agent tensioactif ;
le polymère étant sélectionné parmi le polyvinylpyrrolidone (PVP), l'hydroxypropylcellulose, l'hydroxypropyle méthylcellulose (HPMC) et des mélanges afférents ; et
l'agent tensioactif étant sélectionné parmi les agents non ioniques alcoxylatés, les phénoléthoxylates, les alkylsulfonates, les agents tensioactifs à base d'ester, les agents cationiques et des mélanges afférents ; et dans laquelle :
le médicament antipaludique est sous une forme nano-dispersée qui présente un diamètre de particule de crête mesuré comme il est décrit ici de 20 nm à 800 nm.

5. Procédé pour préparer une composition soluble à l'eau selon l'une quelconque des revendications 1 à 3, lequel comprend les étapes constituées par :
a) la fourniture d'une émulsion qui comprend :
i) une solution du médicament antipaludique non soluble à l'eau dans un solvant non miscible à l'eau pour celui-ci ; et
ii) une solution aqueuse du matériau de support soluble à l'eau ; et
b) le séchage par atomisation de l'émulsion de manière à ôter l'eau et le solvant non miscible à l'eau pour obtenir une nanodispersion sensiblement exempte de solvant du médicament antipaludique dans le matériau de support.

6. Procédé pour préparer une composition soluble à l'eau selon l'une quelconque des revendications 1 à 3, lequel comprend les étapes constituées par :
a) la fourniture d'un mélange qui comprend :
i) au moins un solvant non aqueux miscible à l'eau ;
ii) en option, de l'eau ;
iii) un matériau de support soluble à l'eau qui est soluble dans le mélange de (i) et de (ii) ; et
iv) un médicament antipaludique non soluble dans l'eau qui est soluble dans le mélange de (i) et (ii), et,
b) le séchage par atomisation de la solution de manière à ôter l'eau et le solvant miscible à l'eau pour obtenir une nanodispersion sensiblement exempte de solvant du médicament antipaludique dans le matériau de support.

7. Procédé selon la revendication 5 ou la revendication 6, dans lequel le processus de séchage par atomisation est mis en oeuvre à une température supérieure à 120 degrés Celsius.

8. Procédé selon l'une quelconque des revendications 5 à 7, dans lequel le solvant non aqueux inclut au moins un composant pris parmi le dichlorométhane, le chloroforme, l'éthanol, l'acétone et le sulfoxyde de diméthyle.

9. Procédé pour la préparation d'un médicament pour une utilisation au niveau du traitement ou de la prophylaxie de la malaria, lequel procédé comprend l'étape de préparation d'une composition soluble à l'eau selon l'une quelconque des revendications 1 à 3.
